# EUROPEAN PATENT SPECIFICATION

(11) **EP 2 606 887 B1**
(45) Date of publication and mention of the grant of the patent: **08.08.2018**
(21) Application number: 10856099.6
(22) Date of filing: 28.12.2010
(51) Int. Cl.: A61K 31/404, A61P 1/16

(54) **DIINDOLYLMETHANE, PRECURSOR AND DERIVATIVES THEREOF IN PREPARATION OF MEDICAMENTS FOR TREATING LIVER DISEASES**
DIINDOLYLMETHAN SOWIE VORLÄUFER UND DERIVATE DAVON BEI DER HERSTELLUNG VON MEDIKAMENTEN ZUR BEHANDLUNG VON LEBERERKRANKUNGEN
DIINDOLYLMÉTHANE, SES PRÉCURSEURS ET DÉRIVES UTILISÉS DANS LA PRÉPARATION DE MÉDICAMENTS POUR LE TRAITEMENT DE MALADIES DU FOIE

(30) Priority: 20.08.2010 CN 201010258545
(43) Date of publication of application: 26.06.2013
(73) Proprietor: Nanjing University, Nanjing, Jiangsu 210093 (CN)
(72) Inventor: ZHANG, Junfeng, Nanjing Jiangsu 210093 (CN); DONG, Lei, Nanjing Jiangsu 210093 (CN); HUANG, Zhen, Nanjing Jiangsu 210093 (CN); ZHANG, Zhengping, Nanjing Jiangsu 210093 (CN)
(74) Representative: Kramer Barske Schmidtchen Patentanwälte PartG mbB
(86) International application number: PCT/CN2010/080348
(87) International publication number: WO 2012/022101

(56) References cited:
- WO-A1-2004/093871
- CN-A- 101 428 016
- KR-A- 20090 042 689
- KR-A- 20100 070 664
- US-A1- 2006 111 423
- AIGUO SHEN ET AL: "In vivo study on the protection of indole-3-carbinol (I3C) against the mouse acute alcoholic liver injury by micro-Raman spectroscopy", JOURNAL OF RAMAN SPECTROSCOPY, vol. 40, no. 5, 1 May 2009 (2009-05-01), pages 550-555, XP055107666, ISSN: 0377-0486, DOI: 10.1002/jrs.2163
- SHERTZER H G ET AL: "Protection against carbon tetrachloride hepatotoxicity by pretreatment with indole-3-carbinol", EXPERIMENTAL AND MOLECULAR PATHOLOGY, ACADEMIC PRESS, US, vol. 46, no. 2, 1 April 1987 (1987-04-01), pages 180-189, XP026229133, ISSN: 0014-4800, DOI: 10.1016/0014-4800(87)90064-5 [retrieved on 1987-04-01]
- SHERTZER H G ET AL: "Intervention in free radical mediated hepatotoxicity and lipid peroxidation by indole-3-carbinol", BIOCHEMICAL PHARMACOLOGY, ELSEVIER, US, vol. 37, no. 2, 15 January 1988 (1988-01-15), pages 333-338, XP025541625, ISSN: 0006-2952, DOI: 10.1016/0006-2952(88)90737-X [retrieved on 1988-01-15]
- DONALD S ET AL: "DIETARY AGENT INDOLE-3-CARBINOL PROTECTS FEMALE RATS AGAINST THE HEPATOTOXICITY OF THE ANTITUMOR DRUG ET-743 (TRABECTIDIN) WITHOUT COMPROMISING EFFICACY IN A RAT MAMMARY CARCINOMA", INTERNATIONAL JOURNAL OF CANCER, JOHN WILEY & SONS, INC, US, vol. 111, no. 6, 20 May 2004 (2004-05-20), pages 961-967, XP008044747, ISSN: 0020-7136, DOI: 10.1002/IJC.20356
- DATABASE BIOSIS [Online] BIOSCIENCES INFORMATION SERVICE, PHILADELPHIA, PA, US; April 2007 (2007-04), WU XIAO-QIAN ET AL: "Effects of indole-3-carbinol on hepatic stellate cells activated by acetaldehyde in precision-cut liver slices", XP002721828, Database accession no. PREV200700409522 & ZHONGGUO YAOLIXUE TONGBAO, vol. 23, no. 4, April 2007 (2007-04), pages 441-445, ISSN: 1001-1978
- YU GUO ET AL: "Effect of indole-3-carbinol on ethanol-induced liver injury and acetaldehyde-stimulated hepatic stellate cells activation using precision-cut rat liver slices", CLINICAL AND EXPERIMENTAL PHARMACOLOGY AND PHYSIOLOGY, vol. 37, no. 12, 29 November 2010 (2010-11-29), pages 1107-1113, XP055107669, ISSN: 0305-1870, DOI: 10.1111/j.1440-1681.2010.05450.x
- PING JIE ET AL.: 'Effect of indole-3-carbinol on activation, proliferation and collagen secretion of rat hepatic stellate cells.' CHINESE JOURNAL OF PHARMACOLOGY AND TOXICOLOGY. vol. 21, no. 4, August 2007, page 333, XP008168130
- WU XIAOQIAN ET AL.: 'Effects of indole-3-carbinol on hepatic stellate cells activated by acetaldehyde in precision-cut liver slices.' CHINESE PHARMACOLOGICAL BULLETIN. vol. 23, no. 4, April 2007, pages 441 - 445, XP008168129
- ZHANG CHUN ET AL.: 'Protection of indole-3-carbinol against ethanol-induced hepatotoxicity in precision liver-cut slices of rats.' CHINESE PHARMACOLOGICAL BULLETIN. vol. 20, no. 6, June 2004, pages 660 - 663, XP008168128
- WEI XIAONING ET AL.: 'Molecular Mechanisms of Anti-Fibrotic Effect of Indole-3-Carbinol by Protein Array Assays.' MEDICAL JOURNAL OF WUHAN UNIVERSITY. vol. 31, no. 6, November 2010, pages 709 - 712,738, XP008168158

## Description

### I. Field of the Invention

This invention relates to biopharmaceutical technology, specifically to the application of 3,3'-diindolylmethane derivatives in the preparation of drugs for treating hepatic fibrosis.

### II. Background of the Invention

Hepatic fibrosis results from diffusive deposition of excess extracellular matrix (collagen in particular) in the liver. It is not an independent disease, for many chronic hepatic diseases may lead to hepatic fibrosis. The causes can be infections (such as chronic B, C or D viral hepatitis, schistosomiasis, etc.), congenital metabolic defects (such as hepatolenticular degeneration, hemachromatosis, α1-antitrypsin deficiency, etc.), chemical metabolic defects (chronic alcoholic hepatic disease and chronic drug-induced hepatic diseases) and other diseases like autoimmune hepatitis, primary biliary cirrhosis, and primary sclerosing cholangitis. The clinical symptoms of hepatic fibrosis include tiredness and malaise, inappetence, indigestion and hemorrhage. Cancerization may even occur when the course of fibrosis persists long enough. The existing major medications for treating hepatic fibrosis include glucocorticoids, colchicine and interferon. These traditional medications present such defects as being target nonspecific, strong side effects, relapse after withdrawal and high financial cost.

Recent researches show that, microscopically, the major reason for hepatic fibrosis is activation of hepatic stellate cells (HSCs). The activated HSCs then transform into myofibroblasts. The crucial signal of this process is the expression of α smooth muscle actin (a-SMA), along with high level expression of secretory type I collagen which breaks equilibrium of generation and degeneration of extracellular matrix (ECM), and consequently a large amount of excess collagen deposits in the extracellular area. In the process of fibrosis, hepatic macrophages secrete a large amount of reactive oxygen species (ROS), including superoxide anion (O₂⁻), hydroxy radical (OH⁻) and hydrogen peroxide (H₂O₂). The harm of ROS is twofold: on the one hand, they directly function as signaling molecules that accelerate the proliferation and activation of HSCs leading to hepatic fibrosis; on the other hand, they attack the membranous structures of liver cells and trigger lipid peroxidation chain reactions, which consequently forms a series of lipid peroxides. The lipid peroxides can initiate hepatic fibrosis by interfering with collagen metabolism. 3,3'-Diindolylmethane (DIM) and its precursor, indole-3-carbinol (I3C), are amongst those firstly discovered compounds with anti-carcinogenic functions. Indole-3-carbinol (I3C) is unstable in gastric acid (in vivo). It may form 3,3'-diindolylmethane, an oligomer, through condensation reaction. Currently, though scientific literatures have proved that 3,3'-diindolylmethane can protect the human body from the damage induced by ROS, there is no document involving in its role in treatment of hepatic fibrosis.
US 2006/0111423 A1 discloses the use of diindolylmethane-related indoles and growth factor receptor inhibitors for the treatment of human cytomegalovirus-associated diseases.
A. Shen et al., J. Raman Spectrosc. 2009, 40, 550-555, disclose an *in vivo* study on the protection of indole-3-carbinol (I3C) against the mouse acute alcoholic liver injury by micro-Raman spectroscopy.
H. G. Shertzer et al., Experimental and Molecular Pathology, vol. 46, no. 2, 1987, 180-189, disclose a study on the protection against carbon tetrachloride hepatotoxicity by pretreatment with indole-3-carbinol.
H. G. Shertzer et al., Biochemical Pharmacology, vol. 37, no. 2, 333-338, disclose a study on the intervention in free radical mediated hepatotoxicity and lipid peroxidation by indole-3-carbinol.
S. Donald et al., Int. J. Cancer, 111, 2004, 961-967, disclose that the dietary agent indole-3-carbinol protects female rats against the hepatotoxicity of the antitumor drug ET-743 (Trabectidin) without compromising efficacy in a rat mammary carcinoma.
WO 2004/093871 A1 discloses a method for treating a cancer in a patient in need thereof comprising administering to said patient, concurrently or sequentially, a therapeutically effective amount of at least one indoleamine 2,3-dioxygenase (IDO) inhibitor and at least one signal transduction inhibitor (STI).
KR 2010-0070664 A relates to the use of indole-3-carbinole for preventing steatosis or diabetes.
KR 2009-0042689 A relates to a composition comprising 3,3'-diindolylmethan for preventing and treating inflammation diseases.
J. Ping et al., Chinese Journal of Pharmacology and Toxicology, vol. 21, no. 4, 2007, 333, disclose a study on the effect of indole-3-carbinol on activation, proliferation and collagen secretion of rat hepatic stellate cells.
X. Wu et al., Chinese Pharmacological Bulletin, vol. 23, no. 4, 2007, 441-445, disclose a study on the effect of indole-3-carbinol on hepatic stellate cells activated by acetaldehyde in precision-cut liver slices.
C. Zhang et al., Chinese Pharmacological Bulletin, vol. 20, no. 6, 2004, 660-663, disclose a study on the protection of indole-3-carbinol against ethanol-induced hepatotoxicity in precision-cut liver slices of rats.
X. Wei et al., Medical Journal of Wuhan University, vol. 31, no. 6, 2010, 709-712, 738, disclose a study on the molecular mechanisms of anti-fibrotic effect of indole-3-carbinol by protein array assays.

### III. Summary of the Invention

This invention discloses a type of small molecule drugs for blocking the development of hepatic fibrosis through protecting the human body from ROS-induced damage, specifically, application of diindolylmethane derivatives, and the water injection prepared thereby in treatment of hepatic fibrosis.

This invention provides the use of 3,3'-diindolylmethane derivatives sharing the following structure: 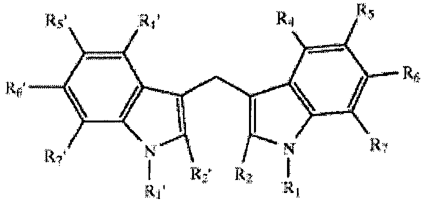 in the preparation of drugs for treating hepatic fibrosis.

The positions R1, R2, R4, R5, R6, R7, R1', R2', R4', R5', R6' and R7' on this structure are hydrogen atoms and at least one halogen. The halogen can be chlorine, bromide or fluorine. The said compounds are also called DIM derivatives or DIM analogs.

In the preferred DIM derivatives, positions R1, R2, R4, R6, R7, R1', R2', R4', R6' and R7' are hydrogen atoms; positions R5 and R5' are halogens that can be chlorine, bromide or fluorine. Accordingly, the preferred DIM derivatives include 5,5'-dichloro-diindolylmethane, 5,5'-dibromo-diindolylmethane, and 5,5'-difluoro-diindolylmethane.

The probably easiest method to obtain the said compounds is to synthesize I3C substituted derivatives with indole substituents that are widely available in the market, and DIM derivatives can be prepared through condensation of indole substituents and methanol. However, the disadvantage of the said method is that the separation and purification of the byproducts emerged during the condensation process require more complex DIM derivatives. In this invention, the said compounds are prepared via indole-3-acetaldehyde substituted derivatives which can be obtained by means of condensation of an appropriate type of indole derivatives with dimethyl formamide. The said appropriate type of indole derivatives may have substituent groups at positions R1, R2, R4, R5, R6 or R7, including 5-chloro-, 5-bromo- and 5-fluoro-. By means of an appropriate type of alcohols, for example, carbinol, and sodium borohydride, the aldehyde groups of the indole-3-acetaldehyde derivatives are reduced to form 13C substituted derivatives. The DIM substituted derivatives can be obtained through condensation of indole-3-carbinol substituted derivatives, which can be realized in phosphate buffer solution (pH value around 5.5).

The agents used according to this invention can be administered orally, intravenously, nasally, rectally or through any other routes that can deliver the effective dosage of bioactive substances. The appropriate dosage is defined as the required final dosage that can be obtained. The said appropriate dosage may vary in treatment of different diseases. The effective dosage of these agents means the dosage that can significantly ameliorate the degree of hepatic fibrosis.

Persons skilled in the art are able to determine the most effective dosage, time and route of administration about the agents used according to this invention. They are able to figure out the metabolism and other pharmacokinetic parameters like distribution, elimination rate of these agents as well.

The bioactive agents used according to this invention can be administered under assistance of carriers or diluents. They can also be administered in combination with other agents, for example, chemotherapeutic drugs or immunoactivators. The embodiments of the said carriers or diluents include any physiological buffer solutions containing water-soluble organic carriers, for example, phosphate buffer solution of cyclodextrin, and other buffer solutions containing appropriate water-soluble organic carriers with pH value around 7.0-7.4. The said appropriate water-soluble organic carriers include but are not limited to cyclodextrin, corn oil, DMSO, capsules, etc.

This invention is illustrated by *in vivo* models of hepatic fibrosis. The animals mentioned herein are but not limited to mice, rat, domesticated animals including but not limited to cats, dogs, cows, sheep, pigs and horses and primates including but not limited to monkeys and human beings. The *in vivo* mouse model of hepatic fibrosis is a widely recognized and accepted model for testing *in vivo* activity of drugs, the results whereof can be used as a reference to the tests on other living beings including the humankind.

The following example is to be regarded as a case to illustrate specific details of this invention. It should not be viewed as to limit the application scope of this invention. Further, it should be noted that only the halogen derivatives as claimed are according to the present invention, and that examples using indole-3-carbinol derivatives are provided for comparative purposes only.

### Application of Indole-3-carbinol derivatives and Diindolylmethane derivatives in the Treatment of Modeled Hepatic Fibrosis:

1. Injecting the phosphate buffer solution of thioacetamide (TTA) into the abdominal cavity of ICR mice so that TTA models of hepatic fibrosis can be established.
2. Dissolving indole-3-carbinol derivatives and diindolylmethane derivatives in corn oil and treating hepatic fibrosis through intragastric administration at the same time of the establishment of TTA models of hepatic fibrosis.
3. Administering TAA, indole-3-carbinol derivatives and diindolylmethane derivatives twice a week for four weeks in succession; killing the mice and taking out the liver for hydroxyproline test and alanine aminotransferase (ALT) test.

### Application of Water Injection of Indole-3-carbinol derivatives and Diindolylmethane derivatives in the Treatment of Modeled Hepatic Fibrosis:

1. Injecting the phosphate buffer solution of thioacetamide (TTA) into the abdominal cavity of ICR mice so that TTA models of hepatic fibrosis can be established.
2. Dissolving indole-3-carbinol derivatives and diindolylmethane derivatives in water and treating hepatic fibrosis through intraperitoneal administration at the same time of the establishment of TTA models of hepatic fibrosis.
3. Administering TAA, indole-3-carbinol derivatives and diindolylmethane derivatives twice a week for four weeks in succession; killing the mice and taking out the liver for hydroxyproline test and alanine aminotransferase (ALT) test.

The beneficial effect of this invention is to provide a new method for treating hepatic fibrosis whereby the water injections of indole-3-carbinol derivatives and diindolylmethane derivatives are used to protect the human body from the damage induced by ROS so that the development of hepatic fibrosis is inhibited or blocked. In addition, the small molecule drugs used in this invention are widely available with fairly cheap price; their stability also guarantees the convenience in storage and transportation.

### IV. Embodiments

### Embodiment 1. Preparation of indole-3-carbinol derivatives and diindolylmethane derivatives

The indole derivatives, for example, 5-methoxyl-, 5-chloro-, 5-bromo-, 5-fluoro-, 5'-methyl-, 5-nitro-, N-methyl-, and 2-methyl-indole can be obtained through purchase (from, for example, Nanjing R&M Pharm-Chem Co., Ltd.); the substituted I3C derivatives can be obtained through reducing the aldehyde groups of indole-3-acetaldehyde derivatives. Cooling dimethyl formamide 2.9ml with ice bath until the temperature drops down to 0°C and slowly adding in phosphorus oxychloride 0.86ml (for 30min); dissolving indole derivatives 8.6mmol in dimethyl formamide 1.0ml and then slowly adding the mixture into the said cooled phosphorus oxychloride solution (for 10min); heating the suspension and keeping the temperature at 37°C for 60-90min until the clean yellow solution turns into yellowish mash; adding icy water 1.0ml into the said mash and then slowly adding in potassium hydrate (KOH) solution 10ml (containing KOH 3.75g) for 30min; heating the said mixture to the boiling point and then cooling it down; filtering the said mixture and indole-3-acetaldehyde derivatives are therefore obtained; washing it with water and drying it in the air. The product so obtained is ready for preparation of I3C derivatives.

Dissolving indole-3-acetaldehyde 1.0g in carbinol 5.0ml and keeping adding in solid sodium borohydride till all the aldehyde groups are reduced; adding H₂O 50ml into the reactants and cooling down to 0°C; filtering the mixture and the I3C derivatives are therefore obtained; vacuum drying the said I3C derivatives in the dark.

Adding I3C 1g into phosphate buffer solution (pH 5.5) and agitating the mixture for 6 hours; testing the whole reaction process with thin layer chromatography (TLC); filtering the mixture and the DIM derivatives are therefore obtained; vacuum drying the product and storing it in the dark; the yield is about 80%-90%, which is verified by gas chromatography, liquid chromatography and thin layer chromatography (TLC).

### Embodiment 2. Treatment of TAA-induced model hepatic fibrosis in mice by orally administered I3C derivatives and DIM derivatives

Dissolving I3C, DIM and methoxyl-DIM in corn oil so that 2.0mg/ml stock solution can be obtained for later use; establishing TAA models of hepatic fibrosis in mice with the method commonly adopted in the related literature, namely, picking 40 mice weighing 16-18g and randomly dividing them into four groups: TAA model group, I3C treatment group, DIM treatment group and 5-methoxyl-DIM treatment group; injecting TAA phosphate buffer solution (200mg/kg) into the abdominal cavity of the mice, twice a week; starting the treatment at the same time of the model establishment: administering the correlated drugs intragastrically at the rate of 20mg/kg to the mice in I3C treatment group, DIM treatment group and 5-methoxyl-DIM treatment group, twice a week; in contrast, the mice in the TAA model group are given only corn oil at the same dosage and frequency. Killing all mice of the four groups 4 weeks later and taking out the pathologically changed hepatic tissue for hydroxyproline test and alanine aminotransferase test.

### (1) Hydroxyproline test

Preparing the tissue homogenate of the pathologically changed liver and extracting the supernatant thereof; testing the concentration of hydroxyproline by means of alkaline hydrolysis.

### (2) Results of alanine aminotransferase test

Testing the serum of mice so that the concentration of alanine aminotransferase can be determined.

When hepatic fibrosis is in process, what accumulate in liver are mainly collagen fibers. Since hydroxyproline is specific to collagen fibers, its concentration to some extent reflects the degree of hepatic fibrosis. As is shown in table 1, the oral administration of I3C, DIM and derivatives thereof can effectively relieve hepatic fibrosis as the concentration of hydroxyproline in the treatment groups is significant lower than that of the TAA model group. Alanine aminotransferase is mainly found in the cytoplasm of liver cells; its concentration therein is 1000-3000 times higher than that in serum; when liver cell necrosis occurs, the serum concentration of alanine aminotransferase will increase dramatically; since the large scale necrosis of liver cells marks the late phase of hepatic fibrosis, the concentration of alanine aminotransferase can also be regarded as an indicator of the degree of hepatic fibrosis. As is also shown in table 1, the concentration of alanine aminotransferase in the treatment groups is significant lower than that of the TAA model group. Therefore, I3C, DIM and derivatives thereof can significantly inhibit necrosis of liver cells, slow down the development of hepatic fibrosis and reduce the damage induced by the disease.

**Table 1 Test of Hydroxyproline in Hepatic Tissue and Alanine Aminotransferase in Serum**

| group | TAA model group | I3C treatment group | DIM treatment group | 5-methoxyl-DIM treatment group |
|---|---|---|---|---|
| hydroxyproline (µg/g liver) | 356±43 | 237±11* | 176±21* | 194±17* |
| alanine aminotransferase (U/mgprot) | 372±24 | 245±32* | 204±37* | 236±12* |

| | | | | |
|---|---|---|---|---|
| All data are listed in the form of "mean value ± standard deviation"; the significant differences are verified with ANOVA test. * indicates P≤0.05. | | | | |

### Embodiment 3. Treatment of TAA-induced model hepatic fibrosis in mice by intraperitoneally administered the water injection of I3C derivatives and DIM derivatives

Dissolving I3C, DIM, methoxyl-DIM and cyclodextrin in physiological saline so that 1.0mg/kg stock solution can be obtained for later use; establishing TAA models of hepatic fibrosis with the method adopted in Embodiment 1; on the same day of the model establishment, mice are divided into four groups, namely, TAA model group, I3C treatment group, DIM treatment group, 5-methoxyl-DIM treatment group; starting the treatment in the same week of the model establishment: administering the correlated drugs intraperitoneally at the rate of 20mg/kg to the mice in I3C treatment group, DIM treatment group and 5-methoxyl-DIM treatment group, twice a week; in contrast, the mice in the TAA model group are given only physiological saline at the same dosage and frequency; killing all mice of the four groups 4 weeks later and taking out the pathologically changed hepatic tissue for hydroxyproline test and alanine aminotransferase test. The methods of hydroxyproline test and alanine aminotransferase test are the same with those adopted in Embodiment 1.

As is shown in Table 2, intraperitoneal administration of water injections of I3C, DIM and derivatives thereof can also inhibit the development of hepatic fibrosis in mice.

**Table 2 Test of Hydroxyproline in Hepatic Tissue and Alanine Aminotransferase in Serum**

| group | TAA model group | I3C treatment group | DIM treatment group | 5-methoxyl-DIM treatment group |
|---|---|---|---|---|
| hydroxyproline (µg/g liver) | 363±32 | 253±21* | 195±24* | 203±14* |
| alanine aminotransferase (U/mgprot) | 392±31 | 239±21* | 187±29* | 226±19* |

| | | | | |
|---|---|---|---|---|
| All data are listed in the form of "mean value ± standard deviation"; the significant differences are verified with ANOVA test. * indicates P≤0.05. | | | | |

The said embodiments are to be regarded as cases to illustrate specific details of this invention; they should not be viewed as to limit the application scope of this invention.

## Claims

1. Use of 3,3'-diindolylmethane derivatives in the preparation of drugs for treating hepatic fibrosis; wherein the 3,3'-diindolylmethane derivatives have the following structure: 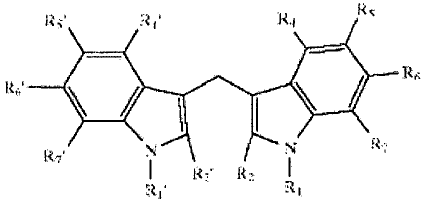 wherein positions R1, R2, R4, R5, R6, R7, R1', R2', R4', R5', R6' and R7' are hydrogen atoms and at least one halogen.

2. The use as is defined in claim 1, wherein the at least one halogen is chlorine, bromide or fluorine.

3. The use as is defined in claim 1, wherein said 3,3'-diindolylmethane derivatives include 5,5'-dichloro-diindolylmethane, 5,5'-dibromo-diindolylmethane, and 5,5'-difluoro-diindolylmethane.

4. The use as is defined in any one of claims 1 to 3, wherein appropriate drug carriers include corn oil, dimethyl sulfoxide, cyclodextrin and collagen capsules.

## Patentansprüche

1. Verwendung von 3,3'-Diindolylmethanderivaten zur Herstellung von Arzneistoffen zur Behandlung von hepatischer Fibrose, wobei die 3,3'-Diindolylmethanderivate die folgende Struktur aufweisen: 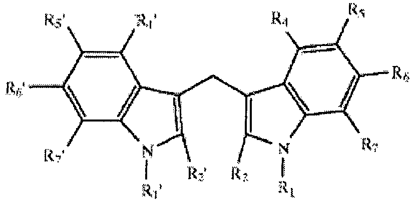 wobei die Positionen R1, R2, R4, R5, R6, R7, R1', R2', R4', R5', R6' und R7' Wasserstoffatome und mindestens ein Halogen sind.

2. Verwendung nach Anspruch 1, wobei das mindestens eine Halogen Chlor, Brom oder Fluor ist.

3. Verwendung nach Anspruch 1, wobei die 3,3'-Diindolylmethanderivate 5,5'-Dichlordiindolylmethan, 5,5'-Dibromdiindolylmethan und 5,5'-Difluordiindolylmethan umfassen.

4. Verwendung nach einem der Ansprüche 1 bis 3, wobei geeignete Arzneistoffträger Maisöl, Dimethylsulfoxid, Cyclodextrin und Kollagenkapseln umfassen.

## Revendications

1. Utilisation de dérivés de 3,3'-di-indolylméthane dans la préparation de médicaments pour le traitement de la fibrose hépatique ; dans laquelle les dérivés de 3,3'-di-indolylméthane ont la structure suivante : 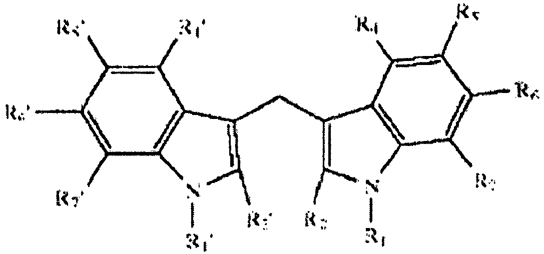 dans laquelle les positions R1, R2, R4, R5, R6, R7, R1', R2', R4', R5', R6' et R7' sont des atomes d'hydrogène et au moins un halogène.

2. Utilisation telle que définie dans la revendication 1, dans laquelle l'au moins un halogène est le chlore, le brome ou le fluor.

3. Utilisation telle que définie dans la revendication 1, dans laquelle lesdits dérivés de 3,3'-di-indolylméthane incluent le 5,5'-dichloro-di-indolylméthane, le 5,5'-dibromo-di-indolylméthane, et le 5,5'-difluoro-di-indolylméthane.

4. Utilisation telle que définie dans l'une quelconque des revendications 1 à 3, dans laquelle les véhicules de médicament appropriés incluent l'huile de maïs, le diméthylsulfoxyde, la cyclodextrine et des capsules de collagène.
